(19) **Europäisches Patentamt / European Patent Office / Office européen des brevets**

(11) **EP 1 429 742 B1**

(12) **EUROPEAN PATENT SPECIFICATION**

(45) Date of publication and mention
of the grant of the patent:
**04.05.2011  Bulletin 2011/18**

(51) Int Cl.:
***A61K 9/28*** (2006.01)

(21) Application number: **02766427.5**

(22) Date of filing: **28.09.2002**

(86) International application number:
**PCT/US2002/031116**

(87) International publication number:
**WO 2003/026615 (03.04.2003 Gazette 2003/14)**

(54) **MODIFIED RELEASE DOSAGE FORMS**

DARREICHUNGSFORMEN ZUR MODIFIZIERTEN FREISETZUNG

FORMES DE DOSAGE A LIBERATION MODIFIEE

(84) Designated Contracting States:
**AT BE BG CH CY CZ DE DK EE ES FI FR GB GR
IE IT LI LU MC NL PT SE SK TR**

(30) Priority: **28.09.2001  US 966939
28.09.2001  US 966509
28.09.2001  US 966497
28.09.2001  US 967414
28.09.2001  US 966450**

(43) Date of publication of application:
**23.06.2004  Bulletin 2004/26**

(73) Proprietor: **McNeil-PPC, Inc.
Skillman,
New Jersey 08558 (US)**

(72) Inventors:
• **LEE, Der-Yang
Flemington, NJ 08822 (US)**
• **LI, Shun-Por
Lansdale, PA 19446 (US)**
• **PARIKH, Narendra
Long Valley, NJ 07853 (US)**

• **MCTEIGUE, Dan
North Wales, PA 19454 (US)**
• **SOWDEN, Harry, S.
Glenside, PA 19038 (US)**
• **THOMAS, Martin
Lake Worth, FL 33467 (US)**
• **WYNN, David
Abington, PA 19001 (US)**

(74) Representative: **Fisher, Adrian John et al
Carpmaels & Ransford
One Southampton Row
London
WC1B 5HA (GB)**

(56) References cited:
**EP-A- 0 279 682     GB-A- 1 144 915
US-A- 5 100 675     US-A- 5 213 808
US-A- 5 415 868     US-A- 5 558 879
US-A- 5 807 579     US-A- 5 824 338**

• **KIBBE A.H.: 'Handbook of Pharmaceutical
Excipients. Third edition.', 2000,
PHARMACEUTICAL PRESS, LONDON * pages
392-393 ***

**Description**

BACKGROUND OF THE INVENTION

1. Field of the Invention

This invention relates to modified release dosage forms such as modified release pharmaceutical compositions. More particularly, this invention relates to modified release dosage forms comprising a molded core, and a shell residing upon at least a portion of the core.

2. Background Information

Modified release pharmaceutical dosage forms have long been used to optimize drug delivery and enhance patient compliance, especially by reducing the number of doses of medicine the patient must take in a day. For this purpose, it is often desirable to modify the rate of release of a drug (one particularly preferred type of active ingredient) from a dosage form into the gastro-intestinal (g.i.) fluids of a patient, especially to slow the release to provide prolonged action of the drug in the body.

The rate at which an orally delivered pharmaceutical active ingredient reaches its site of action in the body depends on a number of factors, including the rate and extent of drug absorption through the g.i. mucosa. To be absorbed into the circulatory system (blood), the drug must first be dissolved in the g.i. fluids. For many drugs, diffusion across the g.i. membranes is relatively rapid compared to dissolution. In these cases, the dissolution of the active ingredient is the rate limiting step in drug absorption, and controlling the rate of dissolution allows the formulator to control the rate of drug absorption into the circulatory system of a patient.

An important objective of modified release dosage forms is to provide a desired blood concentration versus time (pharmacokinetic, or PK) profile for the drug. Fundamentally, the PK profile for a drug is governed by the rate of absorption of the drug into the blood, and the rate of elimination of the drug from the blood. The type of PK profile desired depends, among other factors, on the particular active ingredient, and physiological condition being treated.

One particularly desirable PK profile for a number of drugs and conditions, is one in which the level of drug in the blood is maintained essentially constant (i.e. the rate of drug absorption is approximately equal to the rate of drug elimination) over a relatively long period of time. Such systems have the benefit of reducing the frequency of dosing, improving patient compliance, as well as minimizing side effects while maintaining full therapeutic efficacy. A dosage form which provides a "zero-order," or constant, release rate of the drug is useful for this purpose. Since zero-order release systems are difficult to achieve, systems which approximate a constant release rate, such as for example first-order and square root of time profiles are often used to provide sustained (prolonged, extended, or retarded) release of a drug.

Another particularly desirable PK profile is achieved by a dosage form that delivers a delayed release dissolution profile, in which the release of drug from the dosage form is delayed for a pre-determined time after ingestion by the patient. The delay period ("lag time") can be followed either by prompt release of the active ingredient ("delayed burst"), or by sustained (prolonged, extended, or retarded) release of the active ingredient ("delayed then sustained").

Well known mechanisms by which a dosage form (or drug delivery system) can deliver drug at a controlled rate (e.g. sustained, prolonged, extended or retarded release) include diffusion, erosion, and osmosis.

One classic diffusion-controlled release system comprises a "reservoir" containing the active ingredient, surrounded by a "membrane" through which the active ingredient must diffuse in order to be absorbed into the bloodstream of the patient. The rate of drug release, (dM/dt) depends on the area (A) of the membrane, the diffusional pathlength (1), the concentration gradient (ΔC) of the drug across the membrane, the partition coefficient (K) of the drug into the membrane, and the diffusion coefficient (D):

$$dM/dt = \{ADK\Delta C\} / l$$

Since one or more of the above terms, particularly the diffusional pathlength and concentration gradient tend to be non-constant, diffusion-controlled systems generally deliver a non-constant release rate. In general, the rate of drug release from diffusion-controlled release systems typically follows first order kinetics. One disadvantage of membrane-reservoir type systems is their vulnerability to "dose dumping." The diffusional membrane must remain intact without breach throughout the functional life of the dosage form in order to prevent this occurrence and the possibility of overdose along with the associated toxic side effects. One typical type of diffusional membrane-reservoir systems comprises a compressed tablet core which acts as the reservoir, surrounded by a shell (or coating) which functions as the diffusional membrane. Current core-shell systems are limited by the available methods for manufacturing them, as well as the materials that are suitable for use with the current methods. A shell, or coating, which confers modified release properties is typically applied via conventional methods, such as for example, spray-coating in a coating pan. Pan-coating produces a single shell which essentially surrounds the core. Defects that commonly occur during coating, include "picking," "sticking," and "twinning," all of which result in undesired holes in the coating, which lead to dose dumping. The coating compositions that can be applied via spraying are limited by their viscosity. High viscosity solutions are difficult or impractical to pump and deliver through a spray nozzle. Spray coating methods suffer the further limitations of being time-intensive and costly. Several hours of spraying may be required to spray an effective amount of coating to control the release of an active ingredient. Coating times of 8 to 24 hours are not uncommon.

Another common type of diffusion-controlled release system comprises active ingredient, distributed throughout an insoluble porous matrix through which the active ingredient must diffuse in order to be absorbed into the bloodstream of the patient. The amount of drug (M) released at a given time at sink conditions (i.e. drug concentration at the matrix surface is much greater than drug concentration in the bulk solution), depends on the area (A) of the matrix, the diffusion coefficient (D), the porosity (E) and tortuosity (T) of the matrix, the drug solubility (Cs) in the dissolution medium, time (t) and the drug concentration (Cp) in the dosage form:

$$M \; = \; A \, (DE/T(2Cp - ECs) \, (Cs) \, t)^{1/2}$$

It will be noted in the above relationship that the amount of drug released is generally proportional to the square root of time. Assuming factors such as matrix porosity and tortuosity are constant within the dosage form, a plot of amount of drug released versus the square root of time should be linear. One typical type of diffusional matrix system may be prepared by compression of the active ingredient along with a mixture of soluble and insoluble materials designed to produce a desired porosity and tortuosity as the soluble materials dissolve in the dissolution medium or gastro-intestinal fluids.

A commonly used erosion-controlled release system comprises a "matrix" throughout which the drug is distributed. The matrix typically comprises a material which swells at the surface, and slowly dissolves away layer by layer, liberating drug as it dissolves. The rate of drug release, (dM/dt), in these systems depends on the rate of erosion (dx/dt) of the matrix, the concentration profile in the matrix, and the surface area (A) of the system:

$$dM/dt \; = \; A \, \{dx/dt\} \, \{f(C)\}$$

Again, variation in one or more terms, such as surface area, typically leads to a non-constant release rate of drug. In general, the rate of drug release from erosion-controlled release systems typically follows first order kinetics. One typical method of preparing such eroding matrix systems is by compression of the active ingredient blended with a mixture of compressible excipients comprising water swellable erodible materials which create a temporary barrier as they swell, and allow small amounts of active ingredient to be released as the continuously receding surface layer slowly dissolves in the dissolution medium or gastro-intestinal fluids.

Another type of erosion controlled delivery system employs materials which swell and dissolve slowly by surface erosion to provide a delayed release of pharmaceutical active ingredient. Delayed release is useful, for example in pulsatile or repeat action delivery systems, in which an immediate release dose is delivered, followed by a pre-determined lag time before a subsequent dose is delivered from the system. In these systems, the lag time ($T_1$) depends on the thickness (h) of the erodible layer, and the rate of erosion (dx/dt) of the matrix, which in turn depends on the swelling rate and solubility of the matrix components:

$$T_1 = h \, (dx/dt)$$

The cumulative amount of drug (M) released from these systems at a given time generally follows the equation:

$$M = (dM/dt) \, (t-T_1)$$

where dM/dt is generally described by either the diffusion-controlled or erosion-controlled equations above, and $T_1$ is the lag time.

Modified release dosage forms prepared via compression to obtain either diffusional or eroding matrices are exemplified in U.S. Patent Nos. 5,738,874 and 6,294,200, and WO 99/51209. Compressed dosage forms are limited by the achievable geometry's, as well as the suitable materials for producing them. US 5,213,808 describes a layered dosage form having a pulsatile release profile.

WO 97/49384 describes a hot-melt extrudable mixture of a therapeutic compound and a high molecular weight poly (ethylene oxide). In some embodiments, the formulation further comprises poly (ethylene glycol). The high molecular weight poly (ethylene oxide)s employed have molecular weights ranging from about 1 to about 10 million Daltons. The minimum ratio of high molecular weight poly (ethylene oxide) to active ingredient is 80:20.

The dosage forms of this reference are limited in the amount of active ingredient they can deliver. The maximum amount of active ingredient that may be delivered in the composition is not more that 20 weight percent of the composition. Typical hot-melt systems are additionally limited by high processing temperatures, and are therefore not optimal for delivering low melting, or heat labile active ingredients. Typical hot-melt systems are additionally not optimal for delivering coated particles of active ingredients, due to both the high processing temperatures, and the high shear imparted during processing through extruders or spray nozzles. Typical hot-melt systems are additionally not optimal for applying a coating thereon by conventional methods such as spraying, dipping, or compression.

It would be desirable to have a versatile and cost-effective method for preparing modified release matrix systems, which are not susceptible to dose dumping. It would additionally be desirable to have a method for preparing modified release matrix systems in a variety of shapes, for either functional purposes, e.g. achieving a desired release profile using certain advantageous geometries, or for consumer preference purposes, such as swallowability, dosage form elegance, and product identification and differentiation. It would additionally be desirable to have a controlled release matrix systems capable of delivering a relatively high level of active ingredient in a relatively small dosage form. It would additionally be desirable to have modified release matrix systems for delivering low-melting or heat labile active ingredients. It would additionally be desirable to have modified release matrix systems capable of delivering coated particles of active ingredient. It would additionally be desirable to have a method of applying a shell to a molded core.

It is one object of this invention to provide a dosage form in which at least one active ingredient contained therein exhibits a modified release profile upon contacting of the dosage form with a liquid medium. It is another object of this invention to provide a dosage form in which at least one active ingredient contained therein exhibits a modified release profile upon contacting of the dosage form with a liquid medium. Other objects, features and advantages of the invention will be apparent to those skilled in the art from the detailed description set forth below.

## SUMMARY OF THE INVENTION

The dosage form of this invention comprises: (a) at least one active ingredient; (b) a molded core which is solid at room temperature; and (c) a shell which is in contact with at least a portion of the core, wherein the dosage form provides modified release of the active ingredient upon contacting of the dosage form with a liquid medium.

The molded core comprises one or more active ingredients dispersed in a molded matrix, said matrix comprising a thermal reversible carrier which is polyethylene glycol having a molecular wheight from 100 to 8000 Daltons; and the core comprises one or more release modifying excipients selected from the group consisting of swellable erodible hydrophilic materials, pH-dependent polymers, insoluble edible materials, and pore-formers, and derivatives, copolymers, and combinations thereof.

The core comprises a molded matrix.

The core comprises at least one active ingredient.

The core comprises one or more release-modifying excipients.

The release modifying excipient is selected from the group consisting of swellable erodible hydrophilic materials, pH-dependent polymers, insoluble edible materials, and pore-formers, and derivatives, copolymers, and combinations thereof.

In another embodiment, the core comprises at least 30% of thermal-reversible carrier.

In another embodiment, the core comprises a plurality of particles which comprise at least one active ingredient.

In another embodiment, at least a portion of the particles are coated with a coating comprising 10-100 wt.% of a release-modifying polymer selected from the group consisting of pH-dependent polymers, water-soluble polymer, water-insoluble polymers, and copolymers and derivatives and mixtures thereof.

In another embodiment, the shell has a thickness from about 300 to about 2000 microns.

In another embodiment, the shell has a thickness from about 150 to about 400 microns.

In another embodiment, the weight of the shell is from about 50 to about 400 percent of the weight of the core.

In another embodiment, the weight of the shell is from about 20 to about 100 percent of the weight of the core.

The thermal reversible carrier is polyethylene glycol having a molecular weight from about 100 to about 8000 Daltons.

In another embodiment, the release modifying polymer is shellac.

In another embodiment, the release-modifying excipient is croscarmellose sodium.

BRIEF DESCRIPTION OF THE DRAWINGS

Fig. 1A depicts a cross-sectional side view of one embodiment of the dosage form of this invention.

Fig. 1B depicts a cross-sectional side view of another embodiment of the dosage form of this invention.

Fig. 2 depicts the % release of active ingredient vs. hours measured for the dosage form of Example 1.

DETAILED DESCRIPTION OF THE INVENTION

As used herein, the term "dosage form" applies to any solid object, semi-solid, or liquid composition designed to contain a specific pre-determined amount (i.e. dose) of a certain ingredient, for example an active ingredient as defined below. Suitable dosage forms may be pharmaceutical drug delivery systems, including those for oral administration, buccal administration, rectal administration, topical or mucosal delivery, or subcutaneous implants, or other implanted drug delivery systems; or compositions for delivering minerals, vitamins and other nutraceuticals, oral care agents, flavorants, and the like. Preferably the dosage forms of the present invention are considered to be solid, however they may contain liquid or semi-solid components. In a particularly preferred embodiment, the dosage form is an orally administered system for delivering a pharmaceutical active ingredient to the gastro-intestinal tract of a human.

The dosage forms of the invention exhibit modified release of one or more active ingredients contained therein. One or more active ingredients may be found within the molded matrix and optionally the shell, or coated or uncoated particles distributed therethrough. As used herein, the term "modified release" shall apply to dosage forms, matrices, particles, coatings, portions thereof, or compositions that alter the release of an active ingredient in any manner. The active ingredient or ingredients that are released in a modified manner may be contained within the core and optionally the shell, composition, or portion thereof providing the modification. Types of modified release include controlled, prolonged, sustained, extended, delayed, pulsatile, repeat action, and the like. Suitable mechanisms for achieving these types of modified release include diffusion, erosion, surface area control via geometry and/or impermeable barriers, or other mechanisms known in the art. Moreover, the modified release properties of the dosage form may be achieved through design of the core or a portion thereof, or the shell or a portion thereof, or a combination of these parts of the dosage form.

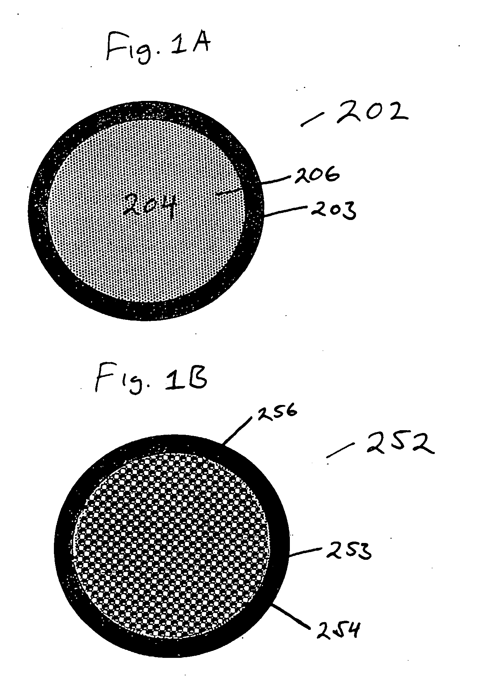

A first embodiment of this invention is depicted in Fig. 1A, which is a cross-sectional side view of a dosage form 202 which comprises a molded core 204 comprising a molded matrix and a shell 203 which is in contact with at least a portion of the core 204. In Fig. 1A the core 204 comprises a plurality of uncoated particles 206 although this is not required in this embodiment of the invention. The active ingredient may be contained within the matrix and, optionally, the uncoated particles (if employed), the shell, or a combination thereof. The dosage form provides modified release of the active ingredient upon contacting of the dosage form with a liquid medium such as water, gastrointestinal fluid and the like. Either the matrix and, optionally, the shell or a combination thereof may provide modified release of the active ingredient.

Another embodiment of this invention is depicted in Fig. 1B, which is a cross-sectional side view of a dosage form 252 which comprises a molded core 254 comprising a molded matrix and a shell 253 which is in contact with at least a portion of the core 254. In Fig. 1B the core 254 comprises a plurality of coated particles 256. The active ingredient is contained within the matrix and, optionally, the coated particles, the shell, or a combination thereof. The dosage form provides modified release of the active ingredient upon contacting of the dosage form with a liquid medium such as water, gastrointestinal fluid and the like. The matrix, and, optionally the coating, the shell or a combination thereof may provide modified release of the active ingredient

The active ingredient employed in the dosage forms of this invention may be found within the core and, optionally, the particles (whether coated or uncoated), the shell or a combination thereof. Suitable active ingredients for use in this invention include for example pharmaceuticals, minerals, vitamins and other nutraceuticals, oral care agents, flavorants and mixtures thereof. Suitable pharmaceuticals include analgesics, anti-inflammatory agents, antiarthritics, anesthetics, antihistamines, antitussives, antibiotics, anti-infective agents, antivirals, anticoagulants, antidepressants, antidiabetic agents, antiemetics, antiflatulents, antifungals, antispasmodics, appetite suppressants, bronchodilators, cardiovascular agents, central nervous system agents, central nervous system stimulants, decongestants, diuretics, expectorants, gastrointestinal agents, migraine preparations, motion sickness products, mucolytics, muscle relaxants, osteoporosis preparations, polydimethylsiloxanes, respiratory agents, sleep-aids, urinary tract agents and mixtures thereof.

Suitable oral care agents include breath fresheners, tooth whiteners, antimicrobial agents, tooth mineralizers, tooth decay inhibitors, topical anesthetics, mucoprotectants, and the like.

Suitable flavorants include menthol, peppermint, mint flavors, fruit flavors, chocolate, vanilla, bubble gum flavors, coffee flavors, liqueur flavors and combinations and the like.

Examples of suitable gastrointestinal agents include antacids such as calcium carbonate, magnesium hydroxide, magnesium oxide, magnesium carbonate, aluminum hydroxide, sodium bicarbonate, dihydroxyaluminum sodium carbonate; stimulant laxatives, such as bisacodyl, cascara sagrada, danthron, senna, phenolphthalein, aloe, castor oil, ricinoleic acid, and dehydrocholic acid, and mixtures thereof; H2 receptor antagonists, such as famotadine, ranitidine, cimetadine, nizatidine; proton pump inhibitors such as omeprazole or lansoprazole; gastrointestinal cytoprotectives, such as sucraflate and misoprostol; gastrointestinal prokinetics, such as prucalopride, antibiotics for H. pylori, such as clarithromycin, amoxicillin, tetracycline, and metronidazole; antidiarrheals, such as diphenoxylate and loperamide; glycopyrrolate; antiemetics, such as ondansetron, analgesics, such as mesalamine.

In one embodiment of the invention, the active ingredient or agent may be selected from bisacodyl, famotadine, ranitidine, cimetidine, prucalopride, diphenoxylate, loperamide, lactase, mesalamine, bismuth, antacids, and pharmaceutically acceptable salts, esters, isomers, and mixtures thereof.

In another embodiment, the active agent is selected from analgesics, antiinflammatories, and antipyretics, e.g. non-steroidal anti-inflammatory drugs (NSAIDs), including propionic acid derivatives, e.g. ibuprofen, naproxen, ketoprofen and the like; acetic acid derivatives, e.g. indomethacin, diclofenac, sulindac, tolmetin, and the like; fenamic acid derivatives, e.g. mefanamic acid, meclofenamic acid, flufenamic acid, and the like; biphenylcarbodylic acid derivatives, e.g. diflunisal, flufenisal, and the like; and oxicams, e.g. piroxicam, sudoxicam, isoxicam, meloxicam, and the like. In a particularly preferred embodiment, the active agent is selected from propionic acid derivative NSAID, e.g. ibuprofen, naproxen, flurbiprofen, fenbufen, fenoprofen, indoprofen, ketoprofen, fluprofen, pirprofen, carprofen, oxaprozin, pranoprofen, suprofen, and pharmaceutically acceptable salts, derivatives, and combinations thereof. In another embodiment of the invention, the active ingredient may be selected from acetaminophen, acetyl salicylic acid, ibuprofen, naproxen, ketoprofen, flurbiprofen, diclofenac, cyclobenzaprine, meloxicam, rofecoxib, celecoxib, and pharmaceutically acceptable salts, esters, isomers, and mixtures thereof.

In another embodiment of the invention, the active ingredient may be selected from pseudoephedrine, phenylpropanolamine, chlorpheniramine, dextromethorphan, diphenhydramine, doxylamine, astemizole, norastemizole, terfenadine, fexofenadine, loratadine, desloratadine, cetirizine, mixtures thereof and pharmaceutically acceptable salts, esters, isomers, and mixtures thereof.

Examples of suitable polydimethylsiloxanes, which include, but are not limited to dimethicone and simethicone, are those disclosed in United States Patent Nos. 4,906,478, 5,275,822, and 6,103,260. As used herein, the term "simethicone" refers to the broader class of polydimethylsiloxanes, including but not limited to simethicone and dimethicone.

The active ingredient or ingredients are present in the dosage form in a therapeutically effective amount, which is an amount that produces the desired therapeutic response upon oral administration and can be readily determined by one skilled in the art. In determining such amounts, the particular active ingredient being administered, the bioavailability characteristics of the active ingredient, the dose regime, the age and weight of the patient, and other factors must be considered, as known in the art. In a preferred embodiment the dosage form comprises one or more active ingredient or ingredients at a combined level of more than about 20 weight percent, e.g. at least about 25 weight percent, or at least about 30 weight percent, or at least about 50 weight percent of the dosage form.

The active ingredient or ingredients may be present in the dosage form in any form. For example, the active ingredient may be dispersed at the molecular level, e.g. melted or dissolved, within the dosage form, or may be in the form of particles, which in turn may be coated or uncoated. If the active ingredient is in form of particles , the particles (whether coated or uncoated) typically have an average particle size of about 1-2000 microns. In one preferred embodiment, such particles are crystals having an average particle size of about 1-300 microns. In another preferred embodiment, the particles are granules or pellets having an average particle size of about 50-2000 microns, preferably about 50-1000 microns, most preferably about 100-800 microns.

The molded matrix of the present invention is made by molding, preferably using a solvent-free process. In a preferred embodiment, the matrix comprises a flowable material. The flowable material may be any edible material that is flowable at a temperature between about 37°C and about 250°C, and that is solid, semi-solid, or can form a gel at a temperature between about -10°C and about 80°C. In a preferred embodiment, the flowable material comprises 10-100% by weight of a thermal reversible carrier having a melting point of less than about 100°C, preferably from about 20 to about 100°C; and optionally up to about 30 weight percent of various adjuvants such as for example plasticizers, gelling agents, colorants, stabilizers, preservatives, and the like as known in the art. The matrix may optionally further comprise up to about 55 weight percent of one or more release-modifying excipients as described below.

In embodiments of this invention in which the matrix comprises 10-100% by weight of a thermal reversible carrier having a melting point of less than about 100°C, such low melting materials may include, for example thermoplastic polyalkalene oxides, low melting hydrophobic materials, thermoplastic polymers, thermoplastic starches, and the like. Preferred low-melting materials may be selected from thermoplastic polymers, thermoplastic polyalkalene oxides, low melting hydrophobic materials, and combinations thereof.

Suitable thermal-reversible carriers for making the molded matrix include are thermoplastic materials typically having a melting point below about 110°C, more preferably between about 20 and about 100°C. The thermoplastic polyalkylene glycols for use as thermal-reversible carriers is polyethylene glycol having molecular weight from about 100 to about 8,000, say about 1000 to about 8,000 Daltons. The matrix composition may also comprise other materials such as release modifying agents, various adjuvants such as for example plasticizers, gelling agents, colorants, stabilizers, preservatives, and the like as known in the art.

Release-modifying moldible excipients for making the molded matrix, or a portion thereof, by molding are swellable erodible hydrophilic materials, pH-dependent polymers, insoluble edible materials, and pore-formers.

Suitable swellable erodible hydrophilic materials for use as release-modifying excipients for making the molded matrix, or a portion thereof, by molding include water swellable cellulose derivatives, polyalkalene glycols, thermoplastic polyalkalene oxides, acrylic polymers, hydrocolloids, clays, gelling starches, and swelling cross-linked polymers, and derivatives, copolymers, and combinations thereof. Examples of suitable water swellable cellulose derivatives include sodium carboxymethylcellulose, cross-linked hydroxypropylcellulose, hydroxypropyl cellulose (HPC), hydroxypropylmethylcellulose (HPMC), hydroxyisopropylcellulose, hydroxybutylcellulose,hydroxyphenylcellulose, hydroxyethylcellulose (HEC), hydroxypentylcellulose, hydroxypropylethylcellulose, hydroxypropylbutylcellulose, hydroxypropylethylcellulose. Examples of suitable polyalkalene glyclols include polyethylene glycol. Examples of suitable thermoplastic polyalkalene oxides include poly (ethylene oxide). Examples of suitable acrylic polymers include potassium methacrylatedivinylbenzene copolymer, polymethylmethacrylate, CARBOPOL (high-molceular weight cross-linked acrylic acid homopolymers and copolymers), and the like. Examples of suitable hydrocolloids include alginates, agar, guar gum, locust bean gum, kappa carrageenan, iota carrageenan, tara, gum arabic, tragacanth, pectin, xanthan gum, gellan gum, maltodextrin, galactomannan, pusstulan, laminarin, scleroglucan, gum arabic, inulin, pectin, gelatin, whelan, rhamsan, zooglan, methylan, chitin, cyclodextrin, chitosan. Examples of suitable clays include smectites such as bentonite, kaolin, and laponite; magnesium trisilicate, magnesium aluminum silicate, and the like, and derivatives and mixtures thereof. Examples of suitable gelling starches include acid hydrolyzed starches, swelling starches such as sodium starch glycolate, and derivatives thereof. Examples of suitable swelling cross-linked polymers include cross-linked polyvinyl pyrrolidone, cross-linked agar, and cross-linked carboxymethylcellose sodium.

Suitable pH-dependent polymers for use as release-modifying excipients for making the molded matrix or a portion thereof by molding include enteric cellulose derivatives, for example hydroxypropyl methylcellulose phthalate, hydroxypropyl methylcellulose acetate succinate, cellulose acetate phthalate; natural resins such as shellac and zein; enteric acetate derivatives such as for example polyvinylacetate phthalate, cellulose acetate phthalate, acetaldehyde dimethylcellulose acetate; and enteric acrylate derivatives such as for example polymethacrylate-based polymers such as poly(methacrylic acid, methyl methacrylate) 1:2, which is commercially available from Rohm Pharma GmbH under the tradename EUDRAGIT S, and poly(methacrylic acid, methyl methacrylate) 1:1, which is commercially available from Rohm Pharma GmbH under the tradename EUDRAGIT L, and the like, and derivatives, salts, copolymers, and combinations thereof.

Suitable insoluble edible materials for use as release-modifying excipients for making the molded matrix, or a portion thereof, by molding include water-insoluble polymers, and low-melting hydrophobic materials. Examples of suitable water-insoluble polymers include ethylcellulose, polyvinyl alcohols, polyvinyl acetate, polycaprolactones, cellulose acetate and its derivatives, acrylates, methacrylates, acrylic acid copolymers; and the like and derivatives, copolymers, and combinations thereof. Suitable low-melting hydrophobic materials include fats, fatty acid esters, phospholipids, and waxes. Examples of suitable fats include hydrogenated vegetable oils such as for example cocoa butter, hydrogenated palm kernel oil, hydrogenated cottonseed oil, hydrogenated sunflower oil, and hydrogenated soybean oil; and free fatty acids and their salts. Examples of suitable fatty acid esters include sucrose fatty acid esters, mono, di, and triglycerides, glyceryl behenate, glyceryl palmitostearate, glyceryl monostearate, glyceryl tristearate, glyceryl trilaurylate, glyceryl myristate, GlycoWax-932, lauroyl macrogol-32 glycerides, and stearoyl macrogol-32 glycerides. Examples of suitable phospholipids include phosphotidyl choline, phosphotidyl serene, phosphotidyl enositol, and phosphotidic acid. Examples of suitable waxes include carnauba wax, spermaceti wax, beeswax, candelilla wax, shellac wax, microcrystalline wax, and paraffin wax; fat-containing mixtures such as chocolate; and the like.

Suitable pore-formers for use as release-modifying excipients for making the molded matrix or a portion thereof by molding include water-soluble organic and inorganic materials. In one embodiment the pore former is hydroxypropylmethylcellulose. Examples of suitable water-soluble organic materials include water soluble polymers including water soluble cellulose derivatives such as hydroxypropylmethylcellulose, and hydroxypropylcellulose; water soluble carbohydrates such as sugars, and starches; water soluble polymers such as polyvinylpyrrolidone and polyethylene glycol, and insoluble swelling polymers such as microcrystalline cellulose. Examples of suitable water soluble inorganic materials include salts such as sodium chloride and potassium chloride and the like and/or mixtures thereof.

Suitable plasticizers for making the molded matrix, or a portion thereof, by molding, include triacetin, acetylated monoglyceride, rape oil, olive oil, sesame oil, acetyltributyl citrate, glycerin sorbitol, diethyloxalate, diethylmalate, diethyl fumarate, dibutyl succinate, diethylmalonate, dioctylphthalate, dibutylsuccinate, triethylcitrate, tributylcitrate, glyceroltributyrate, propylene glycol, polyethylene glycols, hydrogenated castor oil, fatty acids, substituted triglycerides and glycerides, and the like.

The matrix may be in a variety of different shapes. For example, the matrix may be shaped as a polyhedron, such as a cube, pyramid, prism, or the like; or may have the geometry of a space figure with some non-flat faces, such as a cone, truncated cone, cylinder, sphere, torus, or the like. In certain embodiments, the matrix has one or more major faces. For example in certain embodiments matrix surface may have two opposing major faces formed by contact with upper and lower mold surfaces. In such embodiments the core surface may further comprise a "belly-band" located between the two major faces, and formed by contact with the side walls in the mold.

The matrix can be prepared by thermal setting molding using the method and apparatus described in copending U.S. patent application Serial No. 09/966,450, pages 57-63. The matrix is formed by injecting a starting material in flowable form into a molding chamber. The starting material preferably comprises an active ingredient and a thermal setting material at a temperature above the melting point of the thermal setting material but below the decomposition temperature of the active ingredient. The starting material is cooled and solidifies in the molding chamber into a shaped form (i.e., having the shape of the mold).

The matrix can also be prepared by thermal cycle molding using the method and apparatus described in copending U.S. patent application Serial No. 09/966,497, pages 27-51. The matrix is formed by injecting a starting material in flowable form into a heated molding chamber. The starting material preferably comprises an active ingredient and a thermoplastic material at a temperature above the set temperature of the thermoplastic material but below the decomposition temperature of the active ingredient. The starting material is cooled and solidifies in the molding chamber into a shaped form (i.e., having the shape of the mold).

According to these methods, the starting material must be in flowable form. For example, it may comprise solid particles suspended in a molten matrix, for example a polymer matrix. The starting material may be completely molten or in the form of a paste. The starting material may comprise an active ingredient dissolved in a molten material. Alternatively, the starting material may be made by dissolving and/or suspending a solid in a solvent, which solvent is then evaporated from the starting material after it has been molded.

If particles are contained in the matrix, the particles (whether coated or uncoated) typically have an average particle size of about 1-2000 microns. The particles are crystals of the active ingredient or ingredients, and the average particle size is about 1-300 microns. The particles are granules or pellets, and the average particle size is about 50-2000 microns, preferably about 50-1000 microns, most preferably about 100-800 microns.

When uncoated particles are employed, the particles may comprise active ingredient as described herein, or may be inactive particles included for example to provide a visual distinction to the appearance of the dosage form.

When coated particles are employed, the particles may be as described herein, and the particle coating may comprise when coated particles are employed, the particles may be as described herein, and the particle coating may comprise about 10 - 100 weight percent (based on the weight of the coating) of a film former; optionally up to about 50 weight percent based on the weight of the coating of a pore former; and optionally up to about 30 weight percent of various adjuvants or excipients such as plasticizers etc.. The particles may be coated using conventional coating technology which is well known to those skilled in the art including microencapsulation techniques such as coacervation, spray-drying, and fluidized bed coating including tangential spray rotor coating and bottom spray wurster coating. Examples of suitable particle coating methods and materials can be found in United States Patent Nos. 5,286,497; 4,863,742; 4,173,626; 4,980,170; 4,984,240; 5,912,013; 6,270,805; and 6,322,819. Such coated particles may provide controlled release of the active ingredient contained therein in certain embodiments.

Suitable film formers for particle coating include, but are not limited to, film-forming water soluble polymers, film-forming proteins, film-forming water insoluble polymers, and film-forming pH-dependent polymers. In one embodiment, the film-former for particle coating may be selected from cellulose acetate, ammonio methacrylate copolymer type B, shellac, hydroxypropylmethylcellulose, and polyethylene oxide, and combinations thereof.

Suitable film-forming water soluble polymers for particle coating include water soluble vinyl polymers such as polyvinylalcohol; water soluble polycarbohydrates such as hydroxypropyl starch, hydroxyethyl starch, pullulan, methylethyl starch, carboxymethyl starch, pre-gelatinized starches, and film-forming modified starches; water swellable cellulose derivatives such as hydroxypropyl cellulose (HPC), hydroxypropylmethyl cellulose (HPMC), methyl cellulose (MC), hydroxyethylmethylcellulose (HEMC), hydroxybutylmethylcellulose (HBMC), hydroxyethylethylcellulose (HEEC), and hydroxyethylhydroxypropylmethyl cellulose (HEMPMC); water soluble copolymers such as methacrylic acid and methacrylate ester copolymers, polyvinyl alcohol and polyethylene glycol copolymers, polyethylene oxide and polyvinylpyrrolidone copolymers; and derivatives and combinations thereof.

Suitable film-forming proteins may be natural or chemically modified, and include gelatin, whey protein, myofibrillar proteins, coaggulatable proteins such as albumin, casein, caseinates and casein isolates, soy protein and soy protein isolates, zein;; and polymers, derivatives and mixtures thereof.

When the particle coating confers modified release to one or more active ingredients contained in the particle, suitable film formers may be selected from film forming water insoluble polymers; film forming pH-dependent polymers; and copolymers and combinations thereof. When the particle coating functions as a diffusional membrane, the release-modifying particle coating preferably comprises a pore former.

Suitable film forming water insoluble polymers for use in release-modifying particle coatings include for example ethylcellulose, polyvinyl alcohols, polyvinyl acetate, polycaprolactones, cellulose acetate and its derivatives, acrylates, methacrylates, acrylic acid copolymers; and the like and derivatives, copolymers, and combinations thereof.

Suitable film forming pH-dependent polymers for use in release-modifying particle coatings include for example enteric cellulose derivatives, such as for example hydroxypropyl methylcellulose phthalate, hydroxypropyl methylcellulose acetate succinate, cellulose acetate phthalate; natural resins, such as shellac and zein; enteric acetate derivatives such as for example polyvinylacetate phthalate, cellulose acetate phthalate, acetaldehyde dimethylcellulose acetate; and enteric acrylate derivatives such as for example polymethacrylate-based polymers such as poly(methacrylic acid, methyl methacrylate) 1:2, which is commercially available from Rohm Pharma GmbH under the tradename EUDRAGIT S, and poly(methacrylic acid, methyl methacrylate) 1:1, which is commercially available from Rohm Pharma GmbH under the tradename EUDRAGIT L; and the like, and derivatives, salts, copolymers, and combinations thereof.

Suitable pore formers for use in release-modifying particle coatings include water-soluble organic and inorganic materials. In one embodiment the pore former is selected from hydroxypropylcellulose and hydroxypropylmethylcellulose. Examples of suitable water-soluble organic materials include water soluble cellulose derivatives such as hydroxypropylmethylcellulose, and hydroxypropylcellulose; water soluble carbohydrates such as sugars, and starches; water soluble polymers such as polyvinylpyrrolidone and polyethylene glycol, and insoluble swelling polymers such as microcrystalline cellulose. Examples of suitable water soluble inorganic materials include salts such as sodium chloride and potassium chloride and the like and/or mixtures thereof

Examples of suitable adjuvants or excipients for particle coatings include plasticizers, detackifiers, humectants, surfactants, anti-foaming agents, colorants, opacifiers, and the like. Suitable plasticizers for making the core, the shell, or a portion thereof, by molding include, but not be limited to polyethylene glycol; propylene glycol; glycerin; sorbitol; triethyl citrate; tribuyl citrate; dibutyl sebecate; vegetable oils such as castor oil, rape oil, olive oil, and sesame oil; surfactants such as polysorbates, sodium lauryl sulfates, and dioctyl-sodium sulfosuccinates; mono acetate of glycerol; diacetate of glycerol; triacetate of glycerol; natural gums; triacetin; acetyltributyl citrate; diethyloxalate; diethylmalate; diethyl fumarate; diethylmalonate; dioctylphthalate; dibutylsuccinate; glyceroltributyrate; hydrogenated castor oil; fatty acids; substituted triglycerides and glycerides; and the like and/or mixtures thereof. In one embodiment, the plasticizer is triethyl citrate. In certain embodiments, the shell is substantially free of plasticizers, i.e. contains less than about 1%, say less than about 0.01 % of plasticizers.

The dosage form can release one or more active ingredients contained therein in a sustained, extended, prolonged, or retarded manner, more preferably at a substantially constant rate upon contacting of the dosage form with a liquid medium. The molded matrix may function as a diffusional matrix or an eroding matrix. When the molded matrix functions as an eroding matrix from which dispersed active ingredient is liberated in a sustained, extended, prolonged, or retarded manner, the molded matrix preferably comprises a release-modifying moldable excipient selected from swellable erodible hydrophilic materials, pH-dependent polymers, insoluble edible materials, and combinations thereof. When the molded matrix functions as a diffusional matrix through which active ingredient contained therein is liberated in a sustained, extended, prolonged, or retarded manner, the molded matrix preferably comprises a release-modifying excipient selected from combinations of insoluble edible materials and pore formers. Alternately, when the matrix is prepared by solvent-free molding, the thermal-reversible carrier may function by dissolving and forming pores or channels through which the active ingredient may be liberated.

The dosage form can release at least first and second active ingredients contained therein in a sustained, extended, prolonged, or retarded manner. The first and second active ingredients have different unmodified release characteristics; however the dosage form advantageously provides different types of modification to the first and second active ingredients, such that the dissolution profiles of the first and second active ingredients from the dosage form are similar. The dosage form advantageously provides different types of modification to the first and second active ingredients, such that the dissolution profiles of the first and second active ingredients from the dosage form are substantially different, e.g. the first and second active ingredients are released from the dosage for at different rates or times upon contacting of the dosage form with a liquid medium. The first and second active ingredient are both released from the dosage form at a substantially constant rate upon contacting of the dosage form with a liquid medium.

Upon contacting of the dosage form with a liquid medium, a time delay occurs prior to release of at least a portion of one or more active ingredients occurs followed by sustained release of the delayed release active ingredient or ingredients. The time delay is provided by the dissolution of all or a portion of the molded matrix, and the subsequent sustained release is provided by one or more coatings on the particles of active ingredient. The molded matrix preferably comprises a release modifying excipient selected from pH-dependent polymers. The particle coating preferably comprises a release modifying excipient which may be selected from combinations of pore formers and insoluble edible materials; swellable erodible hydrophilic materials; pH-dependent polymers; and combinations thereof.

The dosage form can comprise first and second active ingredients which may be the same or different, and upon contacting of the dosage form with a liquid medium, sustained release of the first active ingredient occurs, followed by sustained release of the second active ingredient. The sustained release of first active ingredient is provided by the controlled dissolution of all or a portion of the molded matrix, and the subsequent sustained release of the second active ingredient is provided by one or more coatings on the particles of active ingredient. The molded matrix preferably comprises a release modifying excipient selected from swellable erodible hydrophilic materials, pH-dependent polymers, insoluble edible materials, and combinations thereof. The particle coating preferably comprises a release modifying excipient which may be selected from combinations of pore formers and insoluble edible materials; swellable erodible hydrophilic materials; pH-dependent polymers, and combinations thereof.

The matrix can comprises a first dose of active ingredient and the particles contained therein comprise a second dose of active ingredient which may be the same or different than the first active ingredient, and upon contacting of the dosage form with a liquid medium, immediate release of the first dose of active ingredient occurs, followed by a lag time, which is in turn followed by delayed release of the second dose active ingredient. The matrix preferably comprises materials which exhibit rapid dissolution in gastro-intestinal fluids. For example the immediate release shell portion or portions may comprise readily soluble materials selected from water soluble or water swellable thermoplastic film formers, water soluble or water swellable thickeners, crystallizable and non-crystallizable carbohydrates. Suitable water soluble or water swellable thermoplastic film formers may be selected from water swellable cellulose derivatives, thermoplastic starches, polyalkalene glycols, polyalkalene oxides, and amorphous sugar glass, and combinations thereof. Suitable film formers may be selected from film forming water soluble polymers such as for example water soluble vinyl polymers, water soluble polycarbohydrates, water swellable cellulose derivatives, and water soluble copolymers; film-forming proteins, and combinations thereof. Suitable thickeners may be selected from gelling polymers or hydrocolloids; gelling starches, and crystallizable carbohydrates, and combinations thereof. Suitable non-crystallizable carbohydrates may be selected from polydextrose, starch hydrolysates, and non-crystallizable sugar alcohols, and combinations thereof. The immediate release matrix will preferably liberate the coated particles of delayed release active ingredient by being breached or dissolved within 30 minutes in 900 ml water or 0.1 N HCl, or phosphate buffer solution at 37°C with stirring by a USP type 2 (Paddle method) at 50 or 100 rpm. The time delay is provided by a coating on the particles containing the second dose of active ingredient. Preferably the delayed release particle coating comprises a release-modifying excipient selected from swellable erodible hydrophilic materials, and pH-dependent polymers, and combinations thereof.

The matrix can comprise a first dose of active ingredient and the particles contained therein comprise a second dose of active ingredient which may be the same or different than the first dose of active ingredient, and upon contacting of the dosage form with a liquid medium, immediate release of the first dose of active ingredient occurs followed by sustained release of the second dose of active ingredient. The matrix preferably comprises materials which exhibit rapid dissolution in gastro-intestinal fluids. For example the immediate release shell portion or portions may comprise readily soluble materials selected from water soluble or water swellable thermoplastic film formers, water soluble or water swellable thickeners, crystallizable and non-crystallizable carbohydrates. Suitable water soluble or water swellable thermoplastic film formers may be selected from water swellable cellulose derivatives, thermoplastic starches, polyalkalene glycols, polyalkalene oxides, and amorphous sugar glass, and combinations thereof. In certain other such embodiments, suitable film formers may be selected from film forming water soluble polymers such as for example water soluble vinyl polymers, water soluble polycarbohydrates, water swellable cellulose derivatives, and water soluble copolymers; film-forming proteins, and combinations thereof. Suitable thickeners may be selected from gelling polymers or hydrocolloids; gelling starches, and crystallizable carbohydrates. Suitable non-crystallizable carbohydrates may be selected from polydextrose, starch hydrolysates, and non-crystallizable sugar alcohols. The immediate release matrix will preferably liberate the coated particles of delayed release active ingredient by being breached or dissolved within 30 minutes in 900 ml water or 0.1 N HCl, or phosphate buffer solution at 37°C with stirring by a USP type 2 (Paddle method) at 50 or 100 rpm. The sustained release is provided by a coating on the particles containing the second dose of active ingredient. Preferably the sustained release particle coating comprises a release-modifying excipient which may be selected from combinations of pore formers and insoluble edible materials; swellable erodible hydrophilic materials; pH-dependent polymers.

Preferably the molded matrix of the present invention is made by injecting the flowable material through an orifice into a mold cavity, then solidifying the flowable material, according to the method set forth herein. When the dosage form comprises particles, the orifice has a diameter greater than the diameter of the particles, e.g. from about 1000 to about 4000 microns, say about 2000 to about 3000 microns. The particles are introduced into the mold cavity in the form of a flowable slurry or suspension in the matrix material. The flowable slurry or suspension may be introduced under pressure through the orifice. The mold assembly may be free of a valve at the injection point. The mold assembly may comprise an elastomeric plug type valve which does not crush the particles upon closing.

Advantageously this method provides a versatile and cost-effective process for preparing the modified release molded matrix systems of the present invention. Advantageously, the method of the present invention may be carried out at relatively low processing temperatures, enabling the incorporation of low melting active ingredients, heat labile active ingredients, and coated particles into molded matrix dosage forms. Advantageously the combination of methods and materials of the present invention enable the incorporation of relatively high levels of active ingredient into the molded matrix dosage form, and enable the production of unique elegant dosage forms with transparent, semitransparent, or translucent matrices.

The shell can contain active ingredient which is released essentially immediately upon ingestion of the dosage form. The shell preferably comprises materials which exhibit rapid dissolution in gastro-intestinal fluids.

The shell functions as a diffusional membrane which contains pores through which fluids can enter the dosage form, and dissolved active ingredient can be released in a sustained, extended, prolonged or retarded manner. The rate of release of active ingredient from the underlying core will depend upon the total pore area in the shell, the pathlength of the pores, and the solubility and diffusivity of the active ingredient (in addition to its rate of release from the core portion itself). When the shell functions as a diffusional membrane, the release of the active ingredient from the dosage form may be described as controlled, prolonged, sustained or extended. The contribution to active ingredient dissolution from the shell may follow zero-order, first-order, or square-root of time kinetics. The diffusional membrane shell portion preferably comprises a release-modifying excipient such as a combination of a pore former and an insoluble edible material such as for example a film forming water insoluble polymer. Alternately, when the shell is prepared by solvent-free molding, the thermal-reversible carrier may function by dissolving and forming pores or channels through which the active ingredient may be liberated.

The shell can function as an eroding matrix from which active ingredient dispersed in the shell is liberated by the dissolution of successive layers of the shell surface. The rate of active ingredient release will depend on the dissolution rate of the matrix material in the shell. Particularly useful matrix materials for providing surface erosion include those which first absorb liquid, then swell and/or gel prior to dissolving. The eroding matrix shell preferably comprises a swellable erodible hydrophilic material.

The shell can function as a barrier to prevent release therethrough of an active ingredient contained in the underlying core or core portion. Active ingredient is typically released from a portion of the core which is not covered by the barrier shell portion. Such embodiments advantageously allow for control of the surface area for release of the active ingredient. For example, the surface area for release of active ingredient can be maintained substantially constant over time. The release of at least one active ingredient follows substantially zero-order kinetics. The barrier shell portion preferably comprises a water insoluble material such as for example a water insoluble polymer.

The shell can function as a delayed release coating to delay release of an active ingredient which is contained in the core or a portion thereof. The lag-time for onset of active ingredient release may be governed by erosion of the shell or diffusion through the shell, or a combination thereof. The eroding matrix shell preferably comprises a swellable erodible hydrophilic material.

When the shell functions to modify the release of an active ingredient which is contained in the core or the subject shell portion, the thickness of the shell portion is critical to the release properties of the dosage form. Advantageously the dosage forms of the invention can be made with precise control over shell thickness. When the shell functions to modify the release of an active ingredient which is contained in the core or the shell, the shell is made by the thermal cycle or thermal setting injection molding methods and apparatus described herein.

The shell of the present invention may be prepared by molding, using a solvent-free process, or a solvent-based process, and depending on the method used, typically comprises a variety of excipients which are useful for conferring desired properties to the shell. The shell may optionally further comprise one or more active ingredients.

When the shell is prepared using a solvent-free molding process, the shell will typically comprise at least about 30 percent, e.g. at least about 45 percent by weight of a thermal-reversible carrier. The shell may optionally further comprise up to about 55 weight percent of a release-modifying excipient. The shell may optionally further comprise up to about 30 weight percent total of various plasticizers, adjuvants and excipients. When the shell is prepared by solvent-free molding, and functions to delay the release of one or more active ingredients from an underlying core portion, the release modifying excipient is preferably selected from swellable, erodible hydrophilic materials.

When the shell is prepared by a solvent-free molding process, the shell typically has a thickness of about 200 to about 4000 microns, e.g. about 300 to about 2000 microns.

When the shell is prepared by a solvent-free molding process, the flowable starting material may be completely molten or in the form of a paste. The starting material may comprise an active ingredient dissolved in a molten material. The ingredients comprising the starting material are preferably mixed together, and heated to a temperature above the melting temperature of the thermal reversible carrier to produce the flowable starting material.

When the shell is prepared using a solvent-based molding process, the shell will typically comprise at least about 10 weight percent, e.g. at least about 12 weight percent or at least about 15 weight percent or at least about 20 weight percent or at least about 25 weight percent of a film-former. Here, the solvent-molded shell may optionally further comprise up to about 55 weight percent of a release-modifying excipient.

The solvent-molded shell may again also optionally further comprise up to about 30 weight percent total of various plasticizers, adjuvants, and excipients. When the shell is prepared by a solvent-based molding process, the shell typically has a thickness of less than about 800 microns, e.g. about 100 to about 600 microns, e.g. about 150 to about 400 microns.

When the shell is prepared by a solvent-based molding process, the flowable starting material may be made by dissolving and/or suspending a solid in a solvent. The solvent is then evaporated from the starting material after it has been molded.

The ingredients comprising the starting material are preferably mixed together, and optionally heated, to disperse the film former and optional other ingredients to produce the flowable starting material.

The total weight of the shell portion or portions is preferably about 20 percent to about 400 percent of the weight of the core. When the shell portion or portions prepared by a solvent-free molding process, the total weight of the shell portion or portions is typically from about 50 percent to about 400 percent, e.g. from about 75 percent to about 400 percent, or about 100 percent to about 200 percent of the weight of the core. When the shell portion or portions are prepared by a solvent-based molding process, the total weight of the shell portion or portions is typically from about 20 percent to about 100 percent of the weight of the core.

Suitable thermal-reversible carriers for preparing the shell by solvent-free molding typically have a melting point below about 110°C, e.g. from about 20 to about 100°C. Suitable thermal-reversible carriers for preparing the shell by solvent-free molding may be selected from the thermal-reversible carriers listed herein for preparing the core by solvent-free molding. Particularly preferred thermal-reversible carriers for preparing the shell by solvent-free molding may be selected from polyethylene glycol, thermoplastic polyethylene oxide, shellac, and combinations thereof.

Suitable release modifying agents for making the shell portion by solvent-free or solvent-based molding include but are not limited to swellable erodible hydrophilic materials, film-formers, pH dependent polymers, and pore-formers.

Suitable plasticizers for making the shell by solvent-free or solvent-based molding include, but are not limited to polyethylene glycol; propylene glycol; glycerin; sorbitol; triethyl citrate; tribuyl citrate; dibutyl sebecate; vegetable oils such as castor oil, rape oil, olive oil, and sesame oil; surfactants such as polysorbates, sodium lauryl sulfates, and dioctyl-sodium sulfosuccinates; mono acetate of glycerol; diacetate of glycerol; triacetate of glycerol; natural gums; triacetin; acetyltributyl citrate; diethyloxalate; diethylmalate; diethyl fumarate; diethylmalonate; dioctylphthalate; dibutylsuccinate; glyceroltributyrate; hydrogenated castor oil; fatty acids; substituted triglycerides and glycerides; and the like and/or mixtures thereof. In one embodiment, the plasticizer is triethyl citrate. In certain embodiments, the shell is substantially free of plasticizers, i.e. contains less than about 1%, say less than about 0.01 % of plasticizers.

Suitable adjuvants and excipients for making the shell by solvent-free or solvent-based molding include secondary film formers such as for example shellac, secondary gelling agents, such as for example cross-linked carboxymethylcellulose, cross-linked polyvinylpyrrolidone, sodium starch glycolate, and the like, as well as preservatives, high intensity sweeteners such as aspartame, acesulfame potassium, sucralose, and saccharin; flavors, antioxidants, surfactants, and coloring agents, many examples of which are known in the art.

Suitable film-formers for preparing the shell by solvent-based molding include, but are not limited to, film-forming water soluble polymers, film-forming proteins, film-forming water insoluble polymers, and film-forming pH-dependent polymers. In one embodiment, the film-former for making the shell or portion thereof by molding may be selected from cellulose acetate, ammonio methacrylate copolymer type B, shellac, hydroxypropylmethylcellulose, and polyethylene oxide, and combinations thereof.

Suitable film-forming water soluble polymers include water soluble vinyl polymers such as polyvinylalcohol (PVA); water soluble polycarbohydrates such as hydroxypropyl starch, hydroxyethyl starch, pullulan, methylethyl starch, carboxymethyl starch, pre-gelatinized starches, and film-forming modified starches; water swellable cellulose derivatives such as hydroxypropyl cellulose (HPC), hydroxypropylmethyl cellulose (HPMC), methyl cellulose (MC), hydroxyethylmethylcellulose (HEMC), hydroxybutylmethylcellulose (HBMC), hydroxyethylethylcellulose (HEEC), and hydroxyethylhydroxypropylmethyl cellulose (HEMPMC); water soluble copolymers such as methacrylic acid and methacrylate ester copolymers, polyvinyl alcohol and polyethylene glycol copolymers, polyethylene oxide and polyvinylpyrrolidone copolymers; and derivatives and combinations thereof.

Suitable film-forming proteins may be natural or chemically modified, and include gelatin, whey protein, myofibrillar proteins, coaggulatable proteins such as albumin, casein, caseinates and casein isolates, soy protein and soy protein isolates, zein;; and polymers, derivatives and mixtures thereof.

Suitable film-forming water insoluble polymers, include for example ethylcellulose, polyvinyl alcohols, polyvinyl acetate, polycaprolactones, cellulose acetate and its derivatives, acrylates, methacrylates, acrylic acid copolymers; and the like and derivatives, copolymers, and combinations thereof.

Suitable film-forming pH-dependent polymers include enteric cellulose derivatives, such as for example hydroxypropyl methylcellulose phthalate, hydroxypropyl methylcellulose acetate succinate, cellulose acetate phthalate; natural resins, such as shellac and zein; enteric acetate derivatives such as for example polyvinylacetate phthalate, cellulose acetate phthalate, acetaldehyde dimethylcellulose acetate; and enteric acrylate derivatives such as for example polymethacrylate-based polymers such as poly(methacrylic acid, methyl methacrylate) 1:2, which is commercially available from Rohm Pharma GmbH under the tradename, EUDRAGIT S, and poly(methacrylic acid, methyl methacrylate) 1:1, which is commercially available from Rohm Pharma GmbH under the tradename, EUDRAGIT L, and the like, and derivatives, salts, copolymers, and combinations thereof.

One suitable hydroxypropylmethylcellulose compound for use as a thermoplastic film-forming water soluble polymer is HPMC 2910, which is a cellulose ether having a degree of substitution of about 1.9 and a hydroxypropyl molar substitution of 0.23, and containing, based upon the total weight of the compound, from about 29% to about 30% methoxyl groups and from about 7% to about 12% hydroxylpropyl groups. HPMC 2910 is commercially available from the Dow Chemical Company under the tradename METHOCEL E. METHOCEL E5, which is one grade of HPMC-2910 suitable for use in the present invention, has a viscosity of about 4 to 6 cps (4 to 6 millipascal-seconds) at 20°C in a 2% aqueous solution as determined by a Ubbelohde viscometer. Similarly, METHOCEL E6 , which is another grade of HPMC-2910 suitable for use in the present invention, has a viscosity of about 5 to 7 cps (5 to 7 millipascal-seconds) at 20°C in a 2% aqueous solution as determined by a Ubbelohde viscometer. METHOCEL E15, which is another grade of HPMC-2910 suitable for use in the present invention, has a viscosity of about 15000 cps (15 millipascal-seconds) at 20°C in a 2% aqueous solution as determined by a Ubbelohde viscometer. As used herein, "degree of substitution" shall mean the average number of substituent groups attached to a anhydroglucose ring, and "hydroxypropyl molar substitution" shall mean the number of moles of hydroxypropyl per mole anhydroglucose.

One suitable polyvinyl alcohol and polyethylene glycol copolymer is commercially available from BASF Corporation under the tradename KOLLICOAT IR.

As used herein, "modified starches" include starches that have been modified by crosslinking, chemically modified for improved stability or optimized performance, or physically modified for improved solubility properties or optimized performance. Examples of chemically-modified starches are well known in the art and typically include those starches that have been chemically treated to cause replacement of some of its hydroxyl groups with either ester or ether groups. Crosslinking, as used herein, may occur in modified starches when two hydroxyl groups on neighboring starch molecules are chemically linked. As used herein, "pre-gelatinized starches" or "instantized starches" refers to modified starches that have been pre-wetted, then dried to enhance their cold-water solubility. Suitable modified starches are commercially available from several suppliers such as, for example, A.E. Staley Manufacturing Company, and National Starch & Chemical Company. One suitable film forming modified starch includes the pre-gelatinized waxy maize derivative starches that are commercially available from National Starch & Chemical Company under the tradenames PURITY GUM and FILMSET, and derivatives, copolymers, and mixtures thereof. Such waxy maize starches typically contain, based upon the total weight of the starch, from about 0 percent to about 18 percent of amylose and from about 100% to about 88% of amylopectin.

Another suitable film forming modified starch includes the hydroxypropylated starches, in which some of the hydroxyl groups of the starch have been etherified with hydroxypropyl groups, usually via treatment with propylene oxide. One example of a suitable hydroxypropyl starch that possesses film-forming properties is available from Grain Processing Company under the tradename, PURE-COTE B790.

Suitable tapioca dextrins for use as film formers include those available from National Starch & Chemical Company under the tradenames CRYSTAL GUM or K-4484, and derivatives thereof such as modified food starch derived from tapioca, which is available from National Starch and Chemical under the tradename PURITY GUM 40, and copolymers and mixtures thereof.

The shell may be prepared using the molding methods and apparatuses described in copending U.S. patent application Serial No. 09/966,939, pages 27-51 and 57-63. The shell itself may comprise at least one active ingredient.

The shell may be applied to the core in the form of a flowable material using the thermal cycle method and apparatus described in copending U.S. patent application Serial No. 09/966,497, pages 27-51. The shell is applied using a thermal cycle molding module having the general configuration shown in Figure 3 therein. The thermal cycle molding module 200 comprises a rotor 202 around which a plurality of mold units 204 are disposed. The thermal cycle molding module includes a reservoir 206 (see Figure 4 therein) for holding shell flowable material. In addition, the thermal cycle molding module is provided with a temperature control system for rapidly heating and cooling the mold units. Figures 55 and 56 depict the temperature control system 600.

The thermal cycle molding module is preferably of the type shown in Figure 28A of copending U.S. Application Serial No. 09/966,497, comprising a series of mold units 204. The mold units 204 in turn comprise upper mold assemblies 214, rotatable center mold assemblies 212 and lower mold assemblies 210 as shown in Figure 28C. Cores are continuously transferred to the mold assemblies, which then close over the cores. The shell flowable material, which is heated to a flowable state in reservoir 206, is injected into the mold cavities created by the closed mold assemblies. The temperature of the shell flowable material is then decreased, hardening it. The mold assemblies open and eject the coated cores. In one particular embodiment, coating is performed in two steps, each half of the cores being coated separately as shown in the flow diagram of Figure 28B of copending U.S. Application Serial No. 09/966,497 via rotation of the center mold assembly.

The shell can completely surround the core.

At least one active ingredient contained within the dosage form exhibits a delayed burst release profile. By "delayed burst release profile" it is meant that the release of that particular active ingredient from the dosage form is delayed for a pre-determined time after ingestion by the patient, and the delay period ("lag time") is followed by prompt (immediate) release of that active ingredient. At least one shell portion of the present invention provides for the delay period and is preferably substantially free of the active ingredient to be released in a delayed burst manner. The delayed burst active ingredient is typically contained within the corresponding underlying core portion. In these embodiments, the core portion may be prepared by compression or molding, and is formulated for immediate release, as is known in the art, so that the core portion is readily soluble upon contact with the dissolution medium. In such embodiments the core portion preferably comprises a disintegrant, and optionally comprises additional excipients such as fillers or thermoplastic materials selected from water-soluble or low-melting materials, and surfactants or wetting agents. The dissolution of the burst release active ingredient, after the delay period, meets USP specifications for immediate release tablets containing that active ingredient. For example, for acetaminophen tablets, USP 24 specifies that in pH 5.8 phosphate buffer, using USP apparatus 2 (paddles) at 50 rpm, at least 80% of the acetaminophen contained in the dosage form is released therefrom within 30 minutes after dosing, and for ibuprofen tablets, USP 24 specifies that in pH 7.2 phosphate buffer, using USP apparatus 2 (paddles) at 50 rpm, at least 80% of the ibuprofen contained in the dosage form is released therefrom within 60 minutes after dosing. See USP 24, 2000 Version, 19 - 20 and 856 (1999).

At least one active ingredient contained within the dosage form exhibits a delayed and sustained release profile. By "delayed then sustained release profile" it is meant that the release of that particular active ingredient from the dosage form is delayed for a pre-determined time after ingestion by the patient, and the delay period ("lag time") is followed by sustained (prolonged, extended, or retarded) release of that active ingredient. At least one shell portion of the present invention provides for the delay period, arid is preferably substantially free of the active ingredient to be released in a delayed then sustained manner. The delayed then sustained release active ingredient is preferably contained within the corresponding underlying core portion. The core portion may function for example as an eroding matrix or a diffusional matrix, or an osmotic pump. When the core portion functions as a diffusional matrix through which active ingredient is liberated in a sustained, extended, prolonged, or retarded manner, the core portion preferably comprises a release-modifying excipient selected from combinations of insoluble edible materials and pore-formers. Alternately, when the core portion is prepared by molding, the thermal-reversible carrier may function by dissolving and forming pores or channels through which the active ingredient may be liberated. When the core portion functions as an eroding matrix from which dispersed active ingredient is liberated in a sustained, extended, prolonged, or retarded manner, the core portion preferably comprises a release-modifying compressible or moldable excipient selected from swellable erodible hydrophilic materials, pH-dependent polymers, and combinations thereof.

The dosage form can comprise first and second active ingredients which may be the same or different, and upon contacting of the dosage form with a liquid medium, delayed release of the first active ingredient occurs followed by sustained release of the second active ingredient.

The shell can comprise a first active ingredient and the core comprises a second active ingredient (for example, within the matrix or coated or uncoated particles or a combination thereof) which may be the same or different than the first active ingredient, and upon contacting of the dosage form with a liquid medium, immediate release of the first active ingredient occurs followed by delayed release of the second active ingredient.

The shell can comprise a first active ingredient and the core comprises a second active ingredient (for example, within the matrix or coated or uncoated particles or a combination thereof) which may be the same or different than the first active ingredient, and upon contacting of the dosage form with a liquid medium, immediate release of the first active ingredient occurs followed by sustained release of the second active ingredient.

The core or matrix or shell of the present invention, whether prepared by a solvent-free molding process, or by a solvent-based molding process, may be substantially free of pores having a diameter of 0.5-5.0 microns. As used herein, "substantially free" means that the shell portion or portions have a pore volume of less than about 0.02 cc/g, preferably less than about 0.01 cc/g, more preferably less than about 0.005 cc/g in the pore diameter range of 0.5 to 5.0 microns. In contrast, typical compressed materials have pore volumes of more than about 0.02 cc/g in this diameter range. In another embodiment of this invention, the core is a molded core and the core or core portions are substantially free of pores having a diameter of 0.5-5.0 microns.

The pore volume, pore diameter and density may be determined using a Quantachrome Instruments PoreMaster 60 mercury intrusion porosimeter and associated computer software program known as "Porowin." The procedure is documented in the Quantachrome Instruments PoreMaster Operation Manual. The PoreMaster determines both pore volume and pore diameter of a solid or powder by forced intrusion of a non-wetting liquid (mercury), which involves evacuation of the sample in a sample cell (penetrometer), filling the cell with mercury to surround the sample with mercury, applying pressure to the sample cell by: (i) compressed air (up to 50 psi maximum); and (ii) a hydraulic (oil) pressure generator (up to 60000 psi maximum). Intruded volume is measured by a change in the capacitance as mercury moves from outside the sample into its pores under applied pressure. The corresponding pore size diameter (d) at which the intrusion takes place is calculated directly from the so-called "Washburn Equation": $d = -(4\gamma(\cos\theta))/P$ where $\gamma$ is the surface tension of liquid mercury, $\theta$ is the contact angle between mercury and the sample surface and P is the applied pressure.

Equipment used for pore volume measurements:

1. Quantachrome Instruments PoreMaster 60.
2. Analytical Balance capable of weighing to 0.0001g.
3. Desiccator.

Reagents used for measurements:

1. High purity nitrogen.
2. Triply distilled mercury.
3. High pressure fluid (Dila AX, available from Shell Chemical Co.).
4. Liquid nitrogen (for Hg vapor cold trap).
5. Isopropanol or methanol for cleaning sample cells.
6. Liquid detergent for cell cleaning.

Procedure:
The samples remain in sealed packages or as received in the dessicator until analysis. The vacuum pump is switched on, the mercury vapor cold trap is filled with liquid nitrogen, the compressed gas supply is regulated at 55 psi., and the instrument is turned on and allowed a warm up time of at least 30 minutes. The empty penetrometer cell is assembled as described in the instrument manual and its weight is recorded. The cell is installed in the low pressure station and "evacuation and fill only" is selected from the analysis menu, and the following settings are employed:

Fine Evacuation time: 1 min.
Fine Evacuation rate: 10
Coarse Evacuation time: 5 min.

The cell (filled with mercury) is then removed and weighed. The cell is then emptied into the mercury reservoir, and two tablets from each sample are placed in the cell and the cell is reassembled. The weight of the cell and sample are then recorded. The cell is then installed in the low-pressure station, the low-pressure option is selected from the menu, and the following parameters are set:

Mode: Low pressure
Fine evacuation rate: 10
Fine evacuation until: 200$\mu$ Hg
Coarse evacuation time: 10 min.
Fill pressure: Contact +0.1
Maximum pressure: 50
Direction: Intrusion And Extrusion
Repeat: 0
Mercury contact angle; 140
Mercury surface tension: 480

Data acquisition is then begun. The pressure vs. cumulative volume-intruded plot is displayed on the screen. After low-pressure analysis is complete, the cell is removed from the low-pressure station and reweighed. The space above the mercury is filled with hydraulic oil, and the cell is assembled and installed in the high-pressure cavity. The following settings are used:

Mode: Fixed rate
Motor speed: 5
Start pressure: 20
End pressure: 60,000
Direction: Intrusion and extrusion
Repeat: 0
Oil fill length: 5
Mercury contact angle: 140
Mercury surface tension: 480

Data acquisition is then begun and graphic plot pressure vs. intruded volume is displayed on the screen. After the high pressure run is complete, the low-and high-pressure data files of the same sample are merged.
This invention will be illustrated by the following examples, which are not meant to limit the invention in any way.

Example 1

Dosage forms according to the invention, comprising molded cores with shells thereon were made as follows.
The molded cores (Example 1A) were made from the following ingredients:

| Tablet | Trade Name | Manufacturer | Weight % | Mg/Tablet |
|---|---|---|---|---|
| Pseudoephedrine Hydrochloride Crystal | | BASF PharmaChemikalien GmbH & Co., Ludwigshafen/ Rhein | 22.0 | 130 |
| Polyethylene Glycol 3350 | Carbowax® | Union Carbide Corporation, Danbury, CT | 45.0 | 267 |
| Shellac Powder | Regular bleached shellac | Mantrose-Haeuser Company, Atteboro, MA | 7.0 | 42 |
| Croscarmellose Sodium | Ac-Di-Sol® | FINE MUSCLE COORDINATION Corporation, Newark DE | 26.0 | 154 |

Processing Steps: A beaker was submersed in a water bath (Ret digi-visc; Antal-Direct, Wayne, PA) where the water temperature was set at 70°C. Polyethylene glycol (PEG) 3350 was added to the beaker and was mixed with a spatula until all PEG was melted. Shellac powder, screened through a #40 mesh screen, was added to the molten PEG and the combined ingredients were mixed until all powder was dispersed. Croscarmellose sodium was then added followed by mixing for 2 minutes. Pseudoephedrine hydrochloride crystal was added, followed by mixing for 5 minutes. 570 to 610 mg of the molten mixture was added a round, concave lower punch and die unit (0.4375 inch diameter) which was manually joined with the upper punch to form a molded tablet core. The molded tablet core was ejected from the die.
The shells (Example 1B) were made of the following ingredients:

| Shell | Trade Name | Manufacturer | Weight % | Mg/Tablet |
|---|---|---|---|---|
| Polyethylene Glycol 3350 | Carbowax® | Union Carbide Corporation, Danbury, CT | 45.0 | 849 |
| Polyethylene Oxide (MW 200,000) | Polyox® WSR N-80 | Union Carbide Corporation, Danbury, CT | 15.0 | 283 |
| Shellac Powder | Regular bleached shellac | Mantrose-Haeuser Company, Atteboro, MA | 20.0 | 377 |
| Croscarmellose Sodium | Ac-Di-Sol® | FMC Corporation, Newark, DE | 10.0 | 188 |
| Tributyl Citrate | | Morflex, Inc., Greensboro, NC | 10.0 | 188 |

Processing Steps: A beaker was submersed in a water bath (Ret digi-visc; Antal-Direct, Wayne, PA) where the water temperature was set at 70°C. Polyethylene glycol (PEG) 3350 was added to the beaker and was mixed with a spatula until all PEG was melted. Shellac powder, screened through a #40 mesh screen, was added to the molten PEG and the ingredients were mixed until all powder was dispersed. Tributyl citrate was added to the molten PEG mixture, followed by mixing for 1 minute. Polyethylene oxide (MW=200,000) was then added, followed by mixing for 10 minutes. Croscarmellose sodium was added, followed by mixing for 2 minutes.
A laboratory scale thermal cycle molding module was used to apply the shell in two portions onto the core. A first mold assembly comprising a cavity was cycled to hot stage at 85°C for 30 seconds. A first portion of the shell material in flowable form (Example 1B) was added to the cavity. A molded core (Example 1A) was then inserted into the cavity. A blank mold assembly that masked half the core was screwed into the first mold assembly. The joined mold assemblies were cycled to cold stage at 5°C for 60 seconds to harden the shell on the exposed half of the core. The blank mold assembly was removed and the molded core coated with the first shell portion was ejected form the cavity.
A second mold assembly comprising a second cavity was cycled to hot stage at 85°C for 30 seconds. A second portion of the shell material in flowable form (Example 1B) was added to the cavity. The molded core comprising the first shell portion was inserted into the second mold assembly in such a way that the uncoated half of the core (without the first shell portion) was inserted into the second mold cavity. The first mold assembly, which was kept in cold cycle at 5°C, was screwed into the second mold assembly. The second mold assembly was cycled to cold stage at 5°C for 60 seconds to harden a second shell portion on the core. The first mold assembly was removed and the dosage form, a molded core coated with the first and second shell portions (Example 1C), was ejected from the mold assembly. The weight gain of the dosage form due to the first and second shell portions was recorded.

Shell material in flowable form (Example 1B) was added into a flat faced, 0.6875 inch rubber mold and a coated core (Example 1C) was inserted into the mold. Additional shell material was added to fill the mold. The round molded tablet core was removed from the mold after 5 minutes of cooling in the mold. The weight gain of the core due to the shell was recorded.

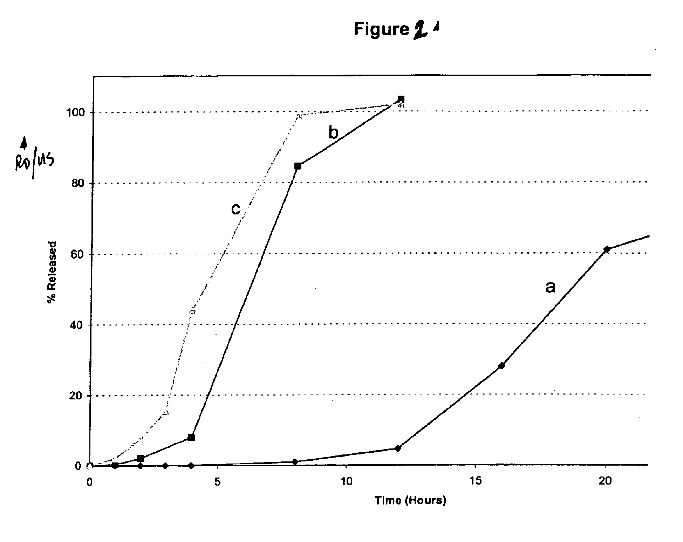

Fig. 2 depicts the % release of active ingredient vs. hours for the dosage form of Example 1 and other dosage forms. More particularly this figure shows the dissolution rate of three different samples of different shell weight gain of the present invention. Curve (a) shows the release rate of pseudoephedrine HCL from the matrix with 314% shell weight gain of this invention. Curve (b) shows the release rate of pseudoephedrine HCL from the matrix with 118% shell weight gain of this invention. Curve (c) shows the release rate of pseudoephedrine HCL from the matrix with 55% shell weight gain of this invention. All curves were derived using the following dissolution analysis: USP Type II apparatus (paddles, 50 RPM) in 0.1 N HCL and pH 5.6 phosphate buffer at 37°C. Samples were tested at 1, 2, 3, 4, 8,12,16,20, and 24 hours for pseudoephedrine HCl. Dissolution samples were analyzed for pseudoephedrine HCl versus a standard prepared at the theoretical concentration for 100% released of each compound. Samples were analyzed using a HPLC equipped with a Waters® 717 Autoinjector and a Waters® 486 UV detector set at a wavelength of 214 nm. The mobile phase was prepared using 55% acetonitrile and 45% 18mM Potassium phosphate buffer. The injection volume was 50 μL with a run time of approximately 8 minutes and a pump flow of 2.0 mL/min. The column used was a Zorbax® 300-SCX (4.6mm x 25 cm).

Example 2

Dosage forms of the invention are made in a continuous process using an apparatus comprising two thermal cycle molding modules linked in series via a transfer device as described at pages 14-16 of copending U.S. Application Serial No. 09/966,939.

The dosage forms comprise a molded core and a shell. The core comprises the ingredients of Example 1A, provided in flowable form as described in Example 1. The shell comprises the ingredients of Example 1B, provided in flowable form as described in Example 1.

The thermal cycle molding modules have the general configuration shown in Figure 3 of copending U.S. Application Serial No. 09/966,497, which depicts a thermal cycle molding module 200 comprising a rotor 202 around which a plurality of mold units 204 are disposed. Each thermal cycle molding module includes its own reservoir 206 (see Figure 4 of copending U.S. Application Serial No. 09/966,497) for holding the core flowable material, and the shell flowable material, respectively. In addition, each thermal cycle molding module is provided with a temperature control system for rapidly heating and cooling the mold units. Figures 55 and 56 of copending U.S. Application Serial No. 09/966,497 depict the temperature control system 600.

The cores are made in a first thermal cycle molding module, which is linked via a transfer device to a second thermal cycle molding module. The first thermal cycle molding module has the specific configuration shown in Figure 26A of copending U.S. Application Serial No. 09/966,497. The first thermal cycle molding module comprises center mold assemblies 212 and upper mold assemblies 214 as shown in Figure 26C, which mate to form mold cavities having the shape of the cores. As rotor 202 rotates, the opposing center and upper mold assemblies close. Core flowable material, which is heated to a flowable state in reservoir 206, is injected into the resulting mold cavities. The temperature of the core flowable material is then decreased, hardening the core flowable material into cores. The mold assemblies open and eject the cores, which are received by the transfer device.

The transfer device has the structure shown as 300 in Figure 3 and described at pages 51-57 of copending U.S. Application Serial No. 09/966,414. It comprises a plurality of transfer units 304 attached in cantilever fashion to a belt 312 as shown in Figures 68 and 69 of copending U.S. Application Serial No. 09/966,414. The transfer device rotates and operates in sync with the thermal cycle molding modules to which it is coupled. Transfer units 304 comprise retainers 330 for holding the cores as they travel around the transfer device.

The transfer device transfers the cores to the second thermal cycle molding module, which applies the shell to the cores. The second thermal cycle molding module is of the type shown in Figure 28A of copending U.S. Application Serial No. 09/966,497. The mold units 204 of the second thermal cycle molding module comprise upper mold assemblies 214, rotatable center mold assemblies 212 and lower mold assemblies 210 as shown in Figure 28C. Cores are continuously transferred to the mold assemblies, which then close over the cores. Shell material, which is heated to a flowable state in reservoir 206, is injected into the mold cavities created by the closed mold assemblies. The temperature of the shell material is then decreased, hardening it. The mold assemblies open and eject the coated cores. Coating is performed in two steps, each half of the cores being coated separately as shown in the flow diagram of Figure 28B of copending U.S. Application Serial No. 09/966,939 via rotation of the center mold assembly.

Although this invention has been illustrated by reference to specific embodiments, it will be apparent to those skilled in the art that various changes and modifications may be made which clearly fall within the scope of this invention.

**Claims**

1. A dosage form for providing modified release of the active ingredient upon contacting of the dosage form with a liquid medium, consisting of:

   (a) a molded core which is solid at room temperature; and
   (b) a shell which is in contact with at least a portion of the molded core,

   wherein:

   the molded core comprises one or more active ingredients dispersed in a molded matrix, said matrix comprising a thermal reversible carrier which is polyethylene glycol having a molecular weight from 100 to 8000 Daltons; and the core comprises one or more release modifying excipients selected from the group consisting of swellable erodible hydrophilic materials, pH-dependent polymers, insoluble edible materials, and pore-formers, and derivatives, copolymers, and combinations thereof.

2. The dosage form of Claim 1, in which the core comprises at least 30% of the thermal reversible carrier.

3. The dosage form of Claim 1, in which the core comprises a plurality of particles which comprise the at least one active ingredient.

4. The dosage form of Claim 3, wherein at least a portion of the particles are coated with a coating comprising 10-100 wt.% of a release-modifying polymer selected from pH-dependent polymers, water-soluble polymers, water insoluble polymers, and copolymers and derivatives and mixtures thereof.

5. The dosage form of Claim 1, wherein the shell has a thickness from 300 to 2000 $\mu$m.

6. The dosage form of Claim 1, wherein the shell has a thickness from 150 to 400 $\mu$m.

7. The dosage form of Claim 1, wherein the weight of the shell is from 50 to 400 percent of the weight of the core.

8. The dosage form of Claim 1, wherein the weight of the shell is from 20 to 100 percent of the weight of the core.

9. The dosage form of Claim 1, wherein the release modifying polymer is shellac.

10. The dosage form of Claim 1, wherein the release modifying excipient is croscarmellose sodium.

**Patentansprüche**

1. Darreichungsform zur Bereitstellung modifizierter Freisetzung des Wirkstoffes bei Inkontaktkommen der Darreichungsform mit einem flüssigen Medium, bestehend aus:

   (a) einem ausgeformten Kern, der bei Raumtemperatur fest ist; und
   (b) einem Mantel, der mit wenigstens einem Abschnitt des ausgeformten Kerns in Kontakt steht,

   wobei:

   der ausgeformte Kern einen oder mehrere Wirkstoffe umfasst, die in einer ausgeformten Matrix dispergiert sind, wobei die Matrix einen thermisch reversiblen Trägerstoff umfasst, der Polyethylenglykol mit einem Molekulargewicht von 100 bis 8000 Daltons ist; und

der Kern einen oder mehrere die Freisetzung modifizierende Hilfsstoffe umfasst, die ausgewählt sind aus der Gruppe, bestehend aus quellbaren erodierbaren hydrophilen Materialien, pH-abhängigen Polymeren, unlöslichen essbaren Materialien und Porenbildnern und Derivaten, Copolymeren und Kombinationen davon.

**2.** Darreichungsform nach Anspruch 1, in der der Kern wenigstens 30% des thermisch reversiblen Trägerstoffes umfasst.

**3.** Darreichungsform nach Anspruch 1, in der der Kern eine Mehrzahl von Teilchen umfasst, die den wenigstens einen Wirkstoff umfassen.

**4.** Darreichungsform nach Anspruch 3, wobei wenigstens ein Teil der Teilchen mit einer Beschichtung beschichtet ist, die 10-100 Gew.-% eines die Freisetzung modifizierenden Polymers umfasst, das ausgewählt ist aus pH-abhängigen Polymeren, wasserlöslichen Polymeren, wasserunlöslichen Polymeren und Copolymeren und Derivaten und Mischungen davon.

**5.** Darreichungsform nach Anspruch 1, wobei der Mantel eine Dicke von 300 bis 2000 $\mu$m besitzt.

**6.** Darreichungsform nach Anspruch 1, wobei der Mantel eine Dicke von 150 bis 400 $\mu$m besitzt.

**7.** Darreichungsform nach Anspruch 1, wobei das Gewicht des Mantels von 50 bis 400 Prozent des Gewichtes des Kerns beträgt.

**8.** Darreichungsform nach Anspruch 1, wobei das Gewicht des Mantels von 20 bis 100 Prozent des Gewichtes des Kerns beträgt.

**9.** Darreichungsform nach Anspruch 1, wobei das die Freisetzung modifizierende Polymer Schellack ist.

**10.** Darreichungsform nach Anspruch 1, wobei der die Freisetzung modifizierende Hilfsstoff Croscarmellose-Natrium ist.

**Revendications**

**1.** Forme de dosage pour entraîner une libération modifiée de l'ingrédient actif lors de la mise en contact de la forme de dosage avec un milieu liquide, consistant en:

(a) un noyau moulé qui est solide à température ambiante; et
(b) une coque qui est en contact avec au moins une portion du noyau moulé, où:

le noyau moulé comprend un ou plusieurs ingrédients actifs dispersés dans une matrice moulée, ladite matrice comprenant un support thermique réversible qui est le polyéthylène glycol ayant une masse moléculaire de 100 à 8000 Daltons; et
le noyau comprend un ou plusieurs excipients de modification de libération sélectionnés dans le groupe consistant en matériaux hydrophiles érodables gonflables, polymères dépendant du pH, matériaux comestibles insolubles et formeurs de pores, et des dérivés, copolymères et combinaisons de ceux-ci.

**2.** Forme de dosage selon la revendication 1, dans laquelle le noyau comprend au moins 30% du support thermique réversible.

**3.** Forme de dosage selon la revendication 1, dans laquelle le noyau comprend une pluralité de particules qui comprennent le au moins un ingrédient actif.

**4.** Forme de dosage selon la revendication 3, dans laquelle au moins une portion des particules sont revêtues d'un revêtement comprenant 10-100% en poids d'un polymère modifiant la libération sélectionné parmi des polymères dépendant du pH, de polymères solubles dans l'eau, de polymères insolubles dans l'eau et de copolymères et dérivés et mélanges de ceux-ci.

**5.** Forme de dosage selon la revendication 1, dans laquelle la coque a une épaisseur de 300 à 2000$\mu$m.

6. Forme de dosage selon la revendication 1, dans laquelle la coque a une épaisseur de 150 à 400$\mu$m.

7. Forme de dosage selon la revendication 1, dans laquelle le poids de la coque représente 50 à 400 pour cent du poids du noyau.

8. Forme de dosage selon la revendication 1, dans laquelle le poids de la coque représente 20 à 100 pour cent en poids du noyau.

9. Forme de dosage selon la revendication 1, dans laquelle le polymère de modification de libération est le shellac.

10. Forme de dosage selon la revendication 1, dans laquelle l'excipient de modification de libération est le sodium de croscarmellose.

Fig. 1A

/ 202

206

203

204

Fig. 1B

256

/ 252

253

254

Figure 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5738874 A
- US 6294200 A
- WO 9951209 A
- US 5213808 A
- WO 9749384 A
- US 4906478 A
- US 5275822 A
- US 6103260 A
- US 966450 A
- US 966497 A
- US 5286497 A
- US 4863742 A
- US 4173626 A
- US 4980170 A
- US 4984240 A
- US 5912013 A
- US 6270805 B
- US 6322819 B
- US 966939 A
- US 966414 A